# EUROPÄISCHE PATENTSCHRIFT

(11) **EP 2 678 306 B1**
(45) Veröffentlichungstag und Bekanntmachung des Hinweises auf die Patenterteilung: **21.06.2017**
(21) Anmeldenummer: 11802039.5
(22) Anmeldetag: 14.12.2011
(51) Int. Cl.: C07C 209/16, C07C 213/02, C07C 227/08, C07D 213/38, C07D 493/04, C07C 211/07, C07C 211/27, C07C 211/35, C07C 211/38, C07C 217/08, C07C 229/08, C07C 229/38

(54) **VERFAHREN ZUR DIREKTEN AMINIERUNG VON ALKOHOLEN MIT AMMONIAK ZU PRIMÄREN AMINEN MITTELS EINES XANTPHOS KATALYSATORSYSTEMS**
PROCESS FOR THE DIRECT AMINATION OF ALCOHOLS USING AMMONIA TO FORM PRIMARY AMINES BY MEANS OF A XANTPHOS CATALYST SYSTEM
PROCÉDÉ POUR L'AMINATION DIRECTE D'ALCOOLS AVEC DE L'AMMONIAQUE EN AMINES PRIMAIRES AU MOYEN D'UN SYSTÈME CATALYTIQUE AU XANTPHOS

(30) Priorität: 21.02.2011 DE 102011004472
(43) Veröffentlichungstag der Anmeldung: 01.01.2014
(73) Patentinhaber: Evonik Degussa GmbH, 45128 Essen (DE)
(72) Erfinder: KLASOVSKY, Florian, 45721 Haltern am See (DE); TACKE, Thomas, 63755 Alzenau (DE); PFEFFER, Jan, Christoph, 45355 Essen (DE); HAAS, Thomas, 48161 Münster (DE); BELLER, Matthias, 18211 Nienhagen (DE); MARTIN, Andreas, 12524 Berlin (DE); DEUTSCH, Jens, 15834 Rangsdorf (DE); KÖCKRITZ, Angela, 12437 Berlin (DE); IMM, Sebastian, 63505 Langenselbold (DE); HABERLAND, Jürgen, 45721 Haltern am See (DE)
(86) Internationale Anmeldenummer: PCT/EP2011/072771
(87) Internationale Veröffentlichungsnummer: WO 2012/113475

(56) Entgegenhaltungen:
- IMM, SEBASTIAN ET AL: "An Efficient and General Synthesis of Primary Amines by Ruthenium-Catalyzed Amination of Secondary Alcohols with Ammonia", ANGEWANDTE CHEMIE, INTERNATIONAL EDITION , 49(44), 8126-8129, S8126/1-S8126/9 CODEN: ACIEF5; ISSN: 1433-7851, 2010, XP002669003,
- IMM S ET AL: "Improved Ruthenium-Catalyzed Amination of Alcohols with Ammonia: Synthesis of Diamines and Amino Esters", ANGEWANDTE CHEMIE. INTERNATIONAL EDITION, WILEY VCH VERLAG, WEINHEIM, Bd. 50, Nr. 33, 8. August 2011 (2011-08-08), Seiten 7599-7603, XP002664616, ISSN: 1433-7851, DOI: 10.1002/ANIE.201103199 [gefunden am 2011-07-05]

## Beschreibung

Die vorliegende Erfindung betrifft ein chemokatalytisches Flüssigphasen-Verfahren zur direkten einstufigen Aminierung von Alkoholen zu primären Aminen mit Ammoniak in hohen Ausbeuten mithilfe eines Katalysatorsystems enthaltend mindestens eine Übergangsmetallverbindung und eine Xantphos-Linganden.

### Stand der Technik

Die Umwandlung von sauerstoffhaltigen in stickstoffhaltige funktionelle Gruppen stellt eine essentielle Transformation für die Synthese einer Vielzahl organischer Verbindungen dar. In Literatur und Technik sind eine Reihe klassischer Methoden bekannt, um die genannte Aufgabe zu lösen.

In der überwiegenden Mehrzahl der Veröffentlichungen wird dabei ein primärer oder sekundärer Alkohol mit einem primären oder sekundären organischen Amin zur Reaktion gebracht. Die Umsetzung eines primären oder sekundären Alkohols mit Ammoniak zu primären Aminen ist dagegen nur unter Anwendung besonderer Verfahrenbedingungen, Katalysatoren und nur mit einigen wenigen Alkoholen beschrieben worden.

Die Herausforderung aller bekannten Verfahren besteht in der Erzielung hoher Selektivitäten zu den primären Aminen, da diese nucleophiler als Ammoniak sind und infolgedessen bevorzugt unter Bildung höherer Amine reagieren können. Während die Umwandlung einer isolierten Hydroxyl- in eine Aminofunktion annähernd thermoneutral verläuft, ist die Bildung sekundärer und tertiärer Amine mit je ca. 30 kJ/mol exotherm und daher auch thermodynamisch gegenüber der Bildung primärer Amine bevorzugt.

### Direkte Aminierung in der Gasphase

Die einstufige direkte Umwandlung einer primären oder sekundären Hydroxylgruppe mit Ammoniak in ein primäres Amin ist im Falle niederer, leicht verdampfbarer Alkohole hauptsächlich auf Gasphasenreaktionen beschränkt. Hierbei wird der entsprechende Alkohol verdampft und unter geeigneten Bedingungen (Druck, Temperatur, Wasserstoff- und ggf. Inertgaspartialdruck) an einem vorwiegend heterogenen Katalysator zur Reaktion gebracht.

Diese Verfahrensweise wird zum Beispiel in den Publikationen US 4314084, US 5530127, US 5932769, FR 1347648, US 3270059, US 4111840, US 4123462, DE 1667193, Fischer et al. (J. Catal., 1999, 182, 289-291) oder Jenzer et al. (Catal. Lett., 1999, 61, 111-114) beschrieben. Nachteil der meisten heterogenkatalysierten Gasphasenvefahren ist die Anwendung hoher Temperaturen (bis zu 400°C) und Drücke (bis zu 300 bar), in deren Folge neben den gewünschten primären Aminen häufig erhebliche Mengen höherer Amine, Alkene und Alkane entstehen. Entsprechend den charakteristischen Druck- und Temperaturbedingungen einer Gasphasenreaktion können mit den genannten Verfahren zudem lediglich solche Substrate in wirtschaftlichen Ausbeuten zu Aminen umgesetzt werden, die sich verlustfrei verdampfen und umsetzen lassen, bzw. wobei sich die Amine verlustfrei kondensieren bzw. resublimieren lassen. Substrate bzw. ihre korrespondierenden Amine, insbesondere solche mit langer Kohlenstoffkette, die unter solchen Bedingungen einer Zersetzung unterliegen, werden in Literatur und Technik daher in Flüssigphasensynthesen umgesetzt.

### Reduktive Aminierung

Dem Fachmann bekannte Verfahren zur Darstellung primärer Amine aus Alkoholen in flüssiger Phase mittels reduktiver Aminierung nutzen eine mehrstufige Vorgehensweise, die mit einem Wechsel des Oxidationszustands des die Hydroxylgruppe tragenden Kohlenstoffatoms einhergehen kann. Hiervon abgegrenzt werden können Verfahren, die unter Beibehaltung der Oxidationsstufe vollzogen werden (direkte Aminierung). Unter Wechsel der Oxidationsstufe des die Hydroxylgruppe tragenden Kohlenstoffatoms (reduktive Aminierung) können Alkohole klassischerweise durch Oxidation zur entsprechenden Carbonylverbindung, nachfolgende Bildung des Imins durch Reaktion mit einer Aminkomponente (primäre, sekundäres Amin oder Ammoniak) und die anschließende homogen- bzw. heterogenkatalysierte Reduktion des Imins mit Wasserstoff dargestellt werden. Die zweistufige Vorgehensweise unter Isolierung der Carbonylverbindung ist jedoch zeit- und kostensintensiv.

### Spezielle Mehrstufenprozesse

Unter Beibehaltung der Oxidationsstufe des die Hydroxylgruppe tragenden Kohlenstoffatoms (direkte Aminierung) können Alkohole durch mehrstufige Substitutionsreaktionen zu Aminen umgesetzt werden. Neben dem finanziellen und zeitlichen Aufwand zur Isolierung der Zwischenstufen wirkt sich bei entsprechenden Verfahren insbesondere der Umgang mit den in diesem Zusammenhang häufig angewandten explosiven und giftigen Aziden sowie die Bildung stöchiometrischer Mengen an Koppelprodukten als nachteilig aus.

### Direkte Flüssigphasenaminierung von Alkoholen

Die direkte einstufige Flüssigphasenaminierung von Alkoholen mit Ammoniak ist in der wissenschaftlichen und Patentliteratur schon länger beschrieben. In einigen Fällen können die beschriebenen Verfahren aufgrund der angewandten Prozessbedingungen nicht eindeutig als Gas- oder Flüssigphasenverfahren eingeordnet werden.

Bei Temperaturen um 170 °C und einem Druck von 200 bar kann nach DE 19507007 Ethanolamin an oxidgeträgerten Ruthenium-Katalysatoren zu Ethylendiamin aminiert werden, wobei die erzielbaren Ausbeuten unter 40 % bleiben, und keine anderen Beispiele beschrieben wurden.

Die Darstellung einwertiger, gegebenenfalls funktionalisierter primärer Amine in hohen Ausbeuten aus den entsprechenden einwertigen, gegebenenfalls funktionalisierten primären Alkoholen werden in den Arbeiten von Milstein et al. beschrieben (Angew. Chem. Int. Ed., 2008, 47, 8661-8664). Hierin wird die direkte einstufige Aminierung z.T. mit Heteroatomen substituierter primärer aliphatischer und benzylischer Alkohole durch 12- bis 36-stündige Umsetzung mit überschüssigem Ammoniak in einem Lösungsmittel bei 7,5 bar und 135-180 °C Reaktionstemperatur beschrieben. Als Katalysator wird der stickstoffhaltige Acridinyl-basierte Pincer-Komplex Carbonylchlorohydrido-[4,5-(di-i-propylphosphinomethylacridino) ruthenium(II)] eingesetzt, und es werden Ausbeuten zwischen 78 und 96 % erzielt.

In WO 2010018570 wird darüber hinaus die Anwendung Chinolinyl-basierter Pincer-Liganden mit vergleichbaren Ausbeuten beschrieben. Der in diesen Veröffentlichungen offenbarte Katalysator bzw. Ligand ist jedoch weder Luft- noch feuchtestabil; der hieraus resultierende erhöhte Prozessaufwand gestattet kein kostengünstiges Herstellungsverfahren für Amine.

Eine Synthese primärer Amine durch Ruthenium-katalysierte Aminierung sekundärer Alkohole mit unterschiedlichen Liganden wird in der Literatur von Imm et al. (S. Imm, S. Bähn, L. Neubert, H. Neumann, M. Beller; Angew. Chem. 2010, 122, 8303-6) beschrieben. Dabei wurde festgestellt, dass sich hohe Ausbeuten zu den Aminierungsprodukten nur dann erhalten lassen, wenn die als Katalysator verwendete Ruthenium-Koordinationsverbindung einen komplizierten, sowohl Phosphor als auch Stickstoff beinhaltenden Liganden enthält. Die besten Ergebnisse für die Aminierung von Cyclohexanol wurden dabei mit dem Liganden CataCXium PCy erhalten (87 % Ausbeute), während rein phosphorhaltige Liganden eine maximale Ausbeute von 20% erlaubten. Jedoch ist auch die mit CataCXium PCy erhältliche Ausbeute noch zu gering für ein wirtschaftliches Verfahren. Imm et al. stellen fest, Xanthphos-Katalysatoren keine Aktivität zeigen.

In gleicher Weise zeigten Pingen et al. (D. Pingen, C. Müller, D. Vogt, Angew. Chem. 2010, 122, 1-5), dass bei der Aminierung von Cyclohexanol sticksstofffreie Phosphanliganden zwar mäßige bis hohe Selektivitäten zu Cyclohexylaminen gestatteten, diese Katalysatoren jedoch nur eine geringe Aktivität aufwiesen (max. Umsatz = 39 %).

Die direkte einstufige Flüssigphasenaminierung funktionalisierter und / oder mehrwertiger Alkohole mit Ammoniak wurde darüber hinaus an heterogenen Katalysatoren beschrieben. Das Ether-Diol Diethylenglycol wurde in DE 3903367 an verschiedenen Zirkondioxid-geträgerten Cu-Co-Ni-Katalysatoren mit flüssigem Ammoniak bei 200 °C in einer 30 bar Wasserstoffatmosphäre aminiert. Als Reaktionsprodukt wurde jedoch in keinem Fall das Ether-Diamin, sondern lediglich die Folgeprodukte Aminoethoxyethanol und Morpholin isoliert.

Unter Verwendung eines Co-Cu-Zn-Katalysators gelingt die Darstellung von Polyetheraminen gemäß US 4153581 jedoch bereits bei 140 °C, ist aber zwingend auf die Anwesenheit von Wasserstoff angewiesen. Allen weiteren in der Literatur bekannten Beispielen zur heterogen- oder homogenkatalysierten Aminierung von Alkoholen bei mäßigen Temperaturen in Anwesenheit von Wasserstoff ((a)Dodecylalkohol in: H. Kimura, Y. Yokota, Y. Sawamoto, Catal. Lett. 2005, 99(3-4), 133-140; (b) Polyvinylalkohol in: G. Vedage, M. Ford, J. Armor, Catalysis of organic reactions 2003, 89, 517-52) oder Abwesenheit von Wasserstoff ((a) 2,5-Dimethoxybenzylalkohol in: P. Likhar, R. Arundhathi, M. Kantam, P. Prathima, Eur. J. Org. Chem. 2009, 5383-59; (b) Hexadecylalkohol in: R. Pruett, M. Keenan, E. Mozelski, US 5103058, 1992.) ist die Tatsache gemeinsam, dass hierbei lediglich nukleophile primäre oder sekundäre Amine mit dem ggf. funktionalisierten Alkohol umgesetzt werden können.

Es ist dem Fachmann daher nicht ersichtlich, dass sich Vertreter dieser Substratklasse mit dem deutlich weniger nukleophilen Ammoniak in hoher Ausbeute zu den entsprechenden primären Aminen umsetzen lassen, zumal in diesem Falle eine Reaktion des gebildeten primären Amins mit noch nicht umgesetztem Alkohol gegenüber der Umsetzung von Ammoniak deutlich bevorzugt ist, was zur Bildung von Nebenprodukten führt.

In verwandten heterogen katalysierten Verfahren werden außerdem Katalysatoren auf Basis von Co-Cr-Mn in Gegenwart von P₂O₅ bei 140-230 °C und 200-300 bar Wasserstoffdruck (DE 1543377), auf Basis von Ni/Al₂O₃ bei 200-230 °C und 15-20 bar Wasserstoffdruck (RO 63243) oder auf Basis von Calcium-Silicoaluminaten bei 260-300 °C und 200 bar Wasserstoffdruck (DE 1278432) beschreiben. Unter vergleichbaren Bedingungen werden Alkohole nach den in DE 19859776 (180-230 °C an Cu-CuO/TiO₂), DE 102006061045 (180-250 °C an Ni-Cu/ZrO₂), DE 102006061042 (180-220 °C an Ni-Cu-Ru/ZrO₂), WO 2008072428 (180-250 °C an Ru/ZrO₂) und WO2007077903 (180-250 °C an Ru/Al₂O₃) beschriebenen Verfahren aminiert; hierbei wird jedoch ebenfalls zusätzlich eine Wasserstoffatmosphäre benötigt.

Die genannten Beispiele verdeutlichen exemplarisch den Bedarf an Verfahren, eine Aktivierung von Alkoholen, insbesondere auch längerkettigen Alkoholen, auch ohne den Einsatz von Wasserstoff zu erreichen und eine Aminierung in hohen Ausbeuten zu realisieren.

In hohen Ausbeuten von bis zu 95,8 % können gemäß DE 1570542 Polyetherdiole wie Polypropylenglycol direkt zu den korrespondierenden Diaminen umgewandelt werden, wenn die Umsetzung bei 240 °C in Gegenwart von Raney-Nickel-Katalysatoren erfolgt. Jedoch ist auch diese Verfahrensweise für die Umsetzung thermolabiler Substrate ungeeignet. Solche thermolabile Substrate sind insbesondere funktionalisierte Alkohole, deren Substituentenmuster bereits im Substrat oder im daraus entstehenden Amin eine inter- bzw. intramolekulare Neben- oder Folgereaktion zulassen, die zum Abbau derselben bzw. zur Bildung unreaktiver höhermolekularer (ggf. polymerer) Produkte führt.

Nach dem geschilderten Stand der Technik ist kein Prozess bekannt, der die direkte einstufige, wasserstofffreie Flüssigphasen-Aminierung von Alkoholen mit Ammoniak zu primären Aminen mittels eines homogenen Katalysatorsystems basierend auf Xantphos und einer Übergangsmetallverbindung bei milden Reaktionsbedingungen und in Abwesenheit von Wasserstoff in hohen Ausbeuten beschreibt. Im Gegenteil beschreibt die herrschende Literaturmeinung, dass solche Katalysatoren keine Reaktivitäten in solchen Aminierungsreaktionen aufweisen.

### Beschreibung der Erfindung

Überraschenderweise und im Gegensatz zur gängigen Literaturmeinung (z. B. Imm et al., Pingen et al.) wurde nun ein Verfahren gefunden, das die direkte Aminierung von Alkoholen mit Ammoniak in Gegenwart eines wie in Anspruch 1 beschriebenen Katalysators und in Abwesenheit von Wasserstoff in hohen Ausbeuten erlaubt.

Gegenstand der vorliegenden Erfindung ist daher ein Verfahren, welches die direkte, homogen katalysierte Flüssigphasenaminierung von Alkoholen, insbesondere mit einer überstöchiometrischen Menge an Ammoniak bezogen auf zu aminierende Hydoxylgruppen, bevorzugt in Abwesenheit von Wasserstoff, gestattet, mithilfe eines Katalysatorsystems enthaltend eine Übergangsmetallverbindung und einen Xantphos-Liganden.

Ein Vorteil des erfindungsgemäßen Verfahrens ist es, dass die bei der Umsetzung ansonsten notwendige Isolierung und Aufreinigung von Zwischenstufen vermieden wird.

Noch ein Vorteil ist es, dass als Liganden für den Katalysator auf stickstoffhaltige Phosphan Liganden, welche luftempfindlich sind, verzichtet werden kann.

Ein weiterer Vorteil ist es, dass auf einfach und in großem Maßstab herstellbare stickstofffreie Phosphanliganden zurückgegriffen werden kann.

Noch ein Vorteil ist es, dass der Einsatz von problematischen Hilfsstoffen wie beispielsweise Aziden vermieden werden kann.

Ein zusätzlicher Vorteil ist weiterhin, dass durch das erfindungsgemäße Verfahren die Bildung von Koppelprodukten entfällt und die Bildung von Nebenprodukten durch geschickte Wahl von Prozessbedingungen und Katalysator auf ein niedriges Maß abgesenkt werden kann.

Vorteilhaft ist es weiterhin, dass der Alkohol in gelöstem Zustand zur Reaktion gebracht wird. Noch ein Vorteil ist, dass die Aminierung des Alkohols ohne eine Isolierung und/oder Aufreinigung von Zwischenstufen oder Intermediaten bewerkstelligt werden kann.

Das erfindungsgemäße Verfahren zur Herstellung von primären Aminen umfasst die Schritte A) Bereitstellung einer Lösung eines Alkohols in einer fluiden, nicht gasförmigen Phase, B) in Kontakt Bringen der Phase mit freiem Ammoniak und/oder mindestens einer Ammoniak freisetzenden Verbindung und eines homogenen Katalysatorsystems enthaltend mindestens einen Xantphos-Liganden und eine Übergangsmetallverbindung, und gegebenenfalls C) Isolierung des in Verfahrensschritt B) gebildeten primären Amins,
Unter dem Begriff "primäres Amin" werden im Zusammenhang mit der vorliegenden Erfindung ebenfalls dessen Salze sowie Mischungen des Amins und/oder seiner Salze verstanden.

Unter dem Begriff "Alkohol" wird im Zusammenhang mit der vorliegenden Erfindung eine organische Verbindung verstanden, die mindestens eine Hydroxylgrupe aufweist. Darüber hinaus kann der so beschriebene Alkohol eine oder mehrere von -OH abweichende funktionelle Gruppen im Molekül tragen.

Unter dem Begriff "Xantphos-Ligand" wird im Zusammenhang mit der vorliegenden Erfindung die Xanthen-basierte Koordinationsverbindung Carbonylchlorohydrido-[9,9-Dimethyl-4,5-bis(diphenylphosphino)-xantheno] ruthenium(II)] verstanden:

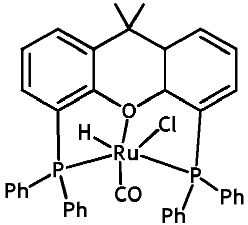

### Carbonylchlorohydrido-[9,9-Dimethyl-4,5-bis(diphenylphosphino)xantheno] ruthenium(II)

Das erfindungsgemäße Verfahren kann für die direkte Aminierung von primären oder sekundären Alkoholen mit Ammoniak zu primären Aminen genutzt werden. Bevorzugt in Verfahrensschritt A) eingesetzte Alkohole sind dadurch gekennzeichnet, dass sie nicht oder nur unzureichend unzersetzt verdampfbar und für eine Gasphasenumsetzung daher nicht geeignet sind. Solche Alkohole sind beispielsweise aliphatische oder aromatische primäre und sekundäre, ggf. mehrwertige Alkohole (wie z.B. die Propanole, Butanole, Pentanole, Hexanole, Octanole, Nonanole, Decanole oder höhere Alkohole), ggf. mehrwertige Benzylalkohole, ggf. mehrwertige cycloaliphatische Alkohole (wie z. B. Cyclopentanol, Cyclohexanol), oder auch andere ggf. mehrwertige hydroxyfunktionalisierte organische Verbindungen mit primärer oder sekundärer OH-Funktion wie z.B. hydroxyfunktionalisierte heterocyclische Verbindungen.

Besonders bevorzugt in Verfahrensschritt A) eingesetzte Alkohole sind ausgewählt aus der Gruppe aliphatischer, linearer ω-Hydroxycarbonsäuren, insbesondere mit einer Kohlenstoffkette umfassend mindestens 8 Kohlenstoffatome, wie zum Beispiel 9-Hydroxynonansäure, 11-Hydroxyundecansäure, 12-Hydroxydodecansäure und die 15-Hydroxypentadecansäure.

Weitere, besonders bevorzugt in Verfahrensschritt A) eingesetzte Alkohole sind aufgrund der milden Reaktionsbedingungen ausgewählt aus der Gruppe der höhermolekularen Alkohole mit einem Molekulargewicht von mindestens 100 g/mol, bevorzugt von mindestens 140 g/mol. Zu typischen Vertretern dieser Gruppe gehören insbesondere die Zuckeralkohole, beispielsweise Isosorbit, Isommanit und deren Derivate ausgewählt aus der Gruppe der Ester- und Etherderviate, Glycoside, Glycale, Desoxyzucker und Glycolipide, und die Polyole, wie z.B. Polyethylenglykole.

Beispielhafte im erfindungsgemäßen Verfahren eingesetzte Alkohol-Konzentrationen bewegen sich im Bereich zwischen 0,1 und 1000 mmol/L, bevorzugt zwischen 0,1 und 100 mmol/L und besonders bevorzugt zwischen 0,1 und 10 mmol/L.

Die im Verfahrensschritt A) eingesetzte fluide Phase kann von einem Lösemittel oder einem unter den Prozessbedingungen in verflüssigter oder überkritischer Form vorliegenden Gas, insbesondere Ammoniak, bzw. Mischungen aus den genannten Komponenten gebildet werden.

Als Lösungsmittel können in diesem Zusammenhang Wasser, oder organische Lösungsmittel oder Mischungen derselben eingesetzt werden, diese Mischungen können eine homogene Lösung oder aber auch eine Emulsion darstellen. Besonders bevorzugt ist der Einsatz mindestens eines organischen Lösungsmittels. Eine nicht als Limitierung anzusehende Auswahl geeigneter organischer Lösemittel umfasst Benzol, Toluol, die Xylol-Isomere, Mesitylen, Dioxan, THF, Dimethoxyethan, Anisol, Cyclohexan, tert.-Butylalkohol und tert.-Amylalkohol.

Als im Verfahrensschritt B) eingesetzter Ammoniak oder eine Ammoniak freisetzende Verbindungen wird im Zusammenhang mit der vorliegenden Erfindung insbesondere auch flüssiger oder überkritischer Ammoniak und/oder eine Lösung von Ammoniumsalzen in einem Lösungsmittel (wie z.B. auch Ammoniumhydroxid in Wasser) verstanden.

Bevorzugt wird in Verfahrensschritt B) als freier Ammoniak gasförmiger oder verflüssigter Ammoniak eingesetzt.

Der Ammoniak in Verfahrensschritt B) wird bezogen auf die Hydroxylgruppen im Alkohol insbesondere in einem molaren Verhältnis von mindestens 5 zu 1, bevorzugt 50 zu 1, besonders bevorzugt 500 zu 1, eingesetzt.

In einer bevorzugten Ausführungsform wird Verfahrensschritt B) bei Überdruck, bezogen auf Atmosphärendruck, durchgeführt. Beispielhafte Drücke im erfindungsgemäßen Verfahren liegen im Bereich zwischen 1 und 1000 bar, bevorzugt zwischen 5 und 500 bar, besonders bevorzugt zwischen 5 und 100 bar und ganz besonders bevorzugt zwischen 5 und 20 bar. Der Druck kann durch Einpressen des Ammoniaks und/oder eines weiteren Gases, insbesondere eines Inertgases wie beispielsweise Stickstoff oder Argon, aufgebaut werden, wobei der Druckaufbau durch Gasmischungen der beiden bevorzugt ist.

Die das erfindungsgemäße Verfahren beschreibenden Temperaturen in Verfahrensschritt B) bewegen sich in einem solchen Bereich, der die aufgrund thermischer Belastung zur Bildung von Nebenprodukten führenden Zersetzungsreaktionen von Alkohol, primärem Amin und allen weiteren im Zuge des Verfahrens auftretenden Intermediaten auf ein Minimum beschränkt. Beispielhaft bewegen sich die Temperaturen in einem Bereich zwischen 80 und 220 °C, bevorzugt zwischen 100 und 200 °C und besonders bevorzugt zwischen 120 und 170 °C, gemessen in der fluiden Phase.

Eine bevorzugte Ausführungsform des Verfahrens ist dadurch gekennzeichnet, dass das Volumenverhältnis der Flüssigphase zu dem der Gasphase im Verfahrensschritt B größer oder gleich 0,05, bevorzugt größer 0,1, insbesondere größer 0,2 ist.

Es ist erfindungsgemäß bevorzugt, dass das Verfahren in Abwesenheit von Wasserstoff durchgeführt wird, wobei unter Abwesenheit von Wasserstoff verstanden wird, dass kein Wasserstoff zusätzlich zur Reaktion zugeführt wird; gegebenenfalls in der Luft enthaltene Spuren von Wasserstoff gelten als "in Abwesenheit von Wasserstoff" im Sinne der vorliegenden Erfindung.

### Beispiele

### Beispiel 1: Direkte einstufige Aminierung der primären OH-Gruppe von 12-Hydroxydodecansäuremethylester mit Ammoniak an einem homogenen, stickstofffreien Ru-Xanthenyl-Katalysator, erfindungsgemäß

In einem 50 mL Hochdruckreaktor wurden 0,23 g 12-Hydroxydodecansäuremethylester (1 mmol), 0,057 g Carbonylchlorohydridotris(triphenylphosphan)ruthenium(II) als Vorläuferverbindung, 0,035 g 9,9-Dimethyl-4,5-bis(diphenylphenylphosphino)xanthen als Ligand und *V* (s*.* Tabelle 1) mL 2-Methyl-2-butanol vorgelegt und im geschlossenen und gasdichten Reaktor bei Raumtemperatur mit Stickstoff gespült. Danach wurden innerhalb von 0,15 Minuten 1,5 mL flüssiger Ammoniak zudosiert und die Reaktionsmischung auf *T*°C (s. Tabelle 1) aufgeheizt, wobei sich ein Druck von bis zu 35 bar einstellt. Nach 20 h Reaktionszeit wurde der Reaktor abgekühlt, entspannt, die Reaktionsmischung in Ethanol aufgenommen und filtriert. Gemäß der in Tabelle 1 aufgeführten NMR-Analysenergebnisse lässt sich das Substrat erfolgreich zum primären Amin umsetzen.

**Tabelle 1**

| Eintrag | V [mL]^{a} | T [°C]^{b} | U [%]^{c} | Aₚᵣᵢₘ [%]^{d} | Aₛₑₖ [%]^{e} |
|---|---|---|---|---|---|
| 1 | 1 | 110 | 45 | 20 | 5 |
| 2 | 1 | 130 | 100 | 30 | 5 |
| 3 | 3 | 130 | 100 | 55 | 8 |

| | | | | | |
|---|---|---|---|---|---|
| a: Volumen 2-Methyl-2-butanol; b: Reaktionstemperatur; c: Umsatz des Substrats; d: Ausbeute zum primären Amin; e: Ausbeute zum sekundären Amin | | | | | |

### Beispiel 2: Direkte einstufige Aminierung des sekundären Alkohols Isosorbid mit Ammoniak an einem homogenen, stickstofffreien Ru-Xanthenyl-Katalysator, erfindungsgemäß

In einem 50 mL Hochdruckreaktorwurden 0,146 g (1 mmol) Isosorbid, 0,057 g (0,06 mmol) Carbonylchlorohydridotris(triphenylphosphan)ruthenium(II), 0,035 g (0,06 mmol) 9,9-Dimethyl-4,5-bis(diphenylphenylphosphino)xanthen und 1 mL tert.-Amylalkohol vorgelegt und im geschlossenen und gasdichten Reaktor bei Raumtemperatur mit Stickstoff gespült. Danach wurden innerhalb von 0,15 Minuten 1,5 mL flüssiger Ammoniak zudosiert und die Reaktionsmischung auf 150 °C aufgeheizt, wobei sich ein Druck von bis zu 35 bar einstellte. Nach 20 h Reaktionszeit wurde der Reaktor abgekühlt, entspannt, die Reaktionsmischung in Ethanol aufgenommen und filtriert. Gemäß NMR-Analytik werden die Alkohol-Gruppen zu 90 % umgesetzt und die korrespondierenden primären Aminogruppen in einer Ausbeute von 70 % erhalten; es wurden keine sekundären Amine nachgewiesen.

### Beispiel 3: Direkte einstufige Aminierung von 2-Octanol mit Ammoniak an einem homogenen Rutheniumkatalysator

Unter einer Argon-Atmosphäre wurden ***mₒ*** g 2-Octanol, ***m_{Ru}*** g [Carbonylchlorohydridotris-(triphenylphosphan)ruthenium(II)] als Katalysator, ***m_{P}*** g 9,9-Dimethyl-4,5-bis(diphenylphosphino)xanthen und ***V_{LM}*** ml 2-Methyl-2-butanol als Lösungsmittel in den Glaseinsatz eines 100 ml Hastelloy-Autoklaven gegeben. Der Autoklav wurde verschlossen, dreimal je 20 bar Argon aufgepresst und entspannt und erneut 15 bzw. 30 bar Argon aufgepresst. Danach wurden ***m_{A}*** g flüssiger Ammoniak in den Autoklaven eingefüllt. Die Reaktionsmischung wurde 10 Minuten bei Raumtemperatur gerührt (600 rpm), anschließend unter Rühren auf 170°C Innentemperatur erhitzt und 48 Stunden bei dieser Temperatur gehalten, wobei sich ein Druck von ***p*** bar einstellte. Nach Abkühlung auf Raumtemperatur, vorsichtigem Entspannen des Ansatzes und dreimaligem Aufpressen von 20 bar Argon mit nachfolgendem Entspannen wurde der Autoklav geöffnet und die Reaktionsmischung mithilfe eines Gaschromatographen analysiert. Reaktionsparameter sowie Umsätze und Selektivitäten zum gewünschten primären Amin 2-Octylamin sind in Tab. 2 wiedergegeben. Die Ergebnisse zeigen, dass die Selektivität zum Zielprodukt sowohl durch Erhöhung des Verhältnisses V_{FI}/_{Gas} als auch durch Erhöhung des Drucks sowie durch gleichzeitige Erhöhung beider Parameter gesteigert werden kann.

**Tabelle 2**

| Nr. | m_{O} [g]¹ | m_{Ru} [g]² | m_{P} [g]³ | V_{LM} [mL]⁴ | m_{A} [g]⁵ | p [bar]⁶ | V_{FI}/V_{Gas} [-]⁷ | U [%]⁸ | S [%]⁹ |
|---|---|---|---|---|---|---|---|---|---|
| 3.1 | 2,6 | 0,58 | 0,35 | 20 | 6,0 | 54 | 1,14 | 99 | 89 |
| 3.2 | 2,6 | 0,58 | 0,,5 | 20 | 6,0 | 89 | 1,14 | 99 | 98 |

| | | | | | | | | | |
|---|---|---|---|---|---|---|---|---|---|
| *1: Masse 2-Octanol; 2: Masse [Carbonylchlorohydridotris-(triphenylphosphan)ruthenium(II)]; 3: Masse Xantphos; 4: Volumen Lösungsmittel; 5: Masse Ammoniak; 6: sich einstellender Druck unter Reaktionsbedingungen; 7: Verhältnis des Flüssigphasenvolumens zum Gasphasenvolumen; 8: Umsatz 2-Octanol; 9: Selektivität zum 2-Octylamin.* | | | | | | | | | |

### Beispiel 4: Direkte einstufige Aminierung von 1-Hexanol mit Ammoniak an einem homogenen Rutheniumkatalysator

Unter Argon-Atmosphäre wurden ***m_{H}*** g 1-Hexanol, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Nach Aufpressen eines Differenzdrucks von weiteren p bar Argon wurde der Reaktor auf 130 °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukt 1-Hexylamin sind in Tab. 3 wiedergegeben. Die Ergebnisse zeigen, dass die Selektivität zum Zielprodukt sowohl durch Erhöhung des Verhältnisses V_{FI}/_{Gas} als auch durch Erhöhung des Drucks sowie durch gleichzeitige Erhöhung beider Parameter gesteigert werden kann.

**Tabelle 3**

| Nr. | m_{H} [g]¹ | m_{Ru} [g]² | m_{P} [g]³ | V_{LM} [mL]⁴ | m_{A} [g]⁵ | p [bar]⁶ | V_{FI}/V_{Gas} [-]⁷ | U [%]⁸ | S [%]⁹ |
|---|---|---|---|---|---|---|---|---|---|
| 4.1 | 0,10 | 0,029 | 0,017 | 1 | 0,3 | 0 | 0,03 | 100 | 31 |
| 4.2 | 0,10 | 0,029 | 0,017 | 1 | 0,3 | 20 | 0,03 | 100 | 37 |
| 4.3 | 0,41 | 0,116 | 0,069 | 4 | 1,2 | 0 | 0,14 | 80 | 50 |
| 4.4 | 0,41 | 0,116 | 0,069 | 4 | 1,2 | 20 | 0,14 | 65 | 48 |

| | | | | | | | | | |
|---|---|---|---|---|---|---|---|---|---|
| *1: Masse 1-Hexanol; 2: Masse [Carbonylchlorohydridotris-(triphenylphosphan)ruthenium(II)]; 3: Masse Xantphos; 4: Volumen Lösungsmittel; 5: Masse Ammoniak; 6: sich einstellender Druck unter Reaktionsbedingungen; 7: Verhältnis des Flüssigphasenvolumens zum Gasphasenvolumen; 8: Umsatz 1-Hexanol; 9: Selektivität zum 1-Hexylamin.* | | | | | | | | | |

### Beispiel 5: Direkte einstufige Aminierung von 12-Hydroxydodecansäuremethylester (Hydroxysäure) mit Ammoniak an einem homogenen Rutheniumkatalysator

Unter Argon-Atmosphäre wurden ***m_{H}*** g 12-Hydroxydodecansäuremethylester, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Nach Aufpressen eines Differenzdrucks von weiteren ***p*** bar Argon wurde der Reaktor auf 130 °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukt 1-Hexylamin sind in Tab. 4 wiedergegeben. Die Ergebnisse zeigen, dass die Selektivität zum Zielprodukt sowohl durch Erhöhung des Verhältnisses V_{FI}/_{Gas} als auch durch Erhöhung des Drucks sowie durch gleichzeitige Erhöhung beider Parameter gesteigert werden kann.

**Tabelle 4**

| Nr. | m_{H} [g]¹ | m_{Ru} [g]² | m_{P} [g]³ | V_{LM} [mL]⁴ | m_{A} [g]⁵ | p [bar]⁶ | V_{FI}/V_{Gas} [-]⁷ | U [%]⁸ | S [%]⁹ |
|---|---|---|---|---|---|---|---|---|---|
| 5.1 | 0,23 | 0,029 | 0,017 | 1 | 0,3 | 0 | 0,04 | 100 | 30 |
| 5.2 | 0,23 | 0,029 | 0,017 | 1 | 0,3 | 20 | 0,04 | 98 | 42 |
| 5.3 | 0,92 | 0,116 | 0,069 | 4 | 1,2 | 0 | 0,16 | 96 | 50 |
| 5.4 | 0,92 | 0,116 | 0,069 | 4 | 1,2 | 20 | 0,16 | 77 | 61 |

| | | | | | | | | | |
|---|---|---|---|---|---|---|---|---|---|
| *1: Masse 12-Hydroxydodecansäuremethylester; 2: Masse [Carbonylchlorohydridotris-(triphenylphosphan)ruthenium(II)]; 3: Masse Xantphos; 4: Volumen Lösungsmittel; 5: Masse Ammoniak; 6: sich einstellender Druck unter Reaktionsbedingungen; 7: Verhältnis des Flüssigphasenvolumens zum Gasphasenvolumen; 8: Umsatz 12-Hydroxydodecansäuremethylester; 9: Selektivität zur 12-Aminododecansäuremethylester.* | | | | | | | | | |

### Beispiel 6: Direkte einstufige Aminierung von Alkoholen und Hydroxysäuren mit Ammoniak an einem homogenen Rutheniumkatalysator und Xantphos bei hohem V_{FI}/V_{Gas} (erfindungsgemäß)

Unter Argon-Atmosphäre wurden ***m_{E}*** g Edukt, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Der Reaktor wird auf ***T*** °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukten sind in Tab. 5 wiedergegeben. Die Ergebnisse zeigen, dass eine Vielzahl unterschiedlicher hydroxyfunktionalisierter Substrate mit der beschriebenen Methode aminiert werden kann.

**Tabelle 5**

| Edukt | m_{E} [g]¹ | m_{Ru} [g]² | m_{P} [g]³ | V_{LM} [mL]⁴ | m_{A} [g]⁵ | T [°C]⁶ | V_{FI}/V_{Gas} [-]⁷ | U [%]⁸ | S [%]⁹ |
|---|---|---|---|---|---|---|---|---|---|
| Tetraethylenglycol | 0,19 | 0,029 | 0,017 | 1 | 1 | 140 | 0,06 | 100 | 97 |
| p-Hydroxymethylbenzylalkohol | 0,14 | 0,029 | 0,017 | 3 | 1 | 150 | 0,10 | 100 | 48 |
| p-Hydroxymethylbenzylalkohol | 0,14 | 0,029 | 0,017 | 5 | 1 | 150 | 0,15 | 100 | 76 |
| m-Hydroxymethylbenzylalkohol | 0,14 | 0,029 | 0,017 | 5 | 1 | 150 | 0,15 | 100 | 70 |
| 1-Octanol | 0,13 | 0,029 | 0,017 | 1 | 1 | 130 | 0,06 | 99 | 53 |
| 1-Octanol | 0,13 | 0,029 | 0,017 | 3 | 1 | 130 | 0,10 | 80 | 79 |
| 1-Octanol | 0,13 | 0,029 | 0,017 | 3 | 1 | 140 | 0,10 | 99 | 80 |
| 2-Phenylethanol | 0,12 | 0,029 | 0,017 | 3 | 1 | 140 | 0,10 | 99 | 94 |
| Benzylalkohol | 0,11 | 0,029 | 0,017 | 3 | 1 | 140 | 0,10 | 100 | 87 |
| 3-Pyridinylmethanol | 0,11 | 0,029 | 0,017 | 3 | 1 | 140 | 0,10 | 100 | 96 |
| 10-Hydroxydecansäure-methylester | 0,20 | 0,029 | 0,017 | 3 | 1 | 130 | 0,10 | 100 | 75 |
| 4-Hydroxymethylbenzoesäuremethylester | 0,17 | 0,029 | 0,017 | 3 | 0,6 | 130 | 0,09 | 100 | 92 |
| Isosorbid | 0,15 | 0,058 | 0,035 | 1 | 0,6 | 150 | 0,04 | 90 | 22 |
| Isosorbid | 0,15 | 0,058 | 0,035 | 1 | 0,6 | 170 | 0,04 | 100 | 96 |
| 1,4-Cyclohexandiol | 0,11 | 0,029 | 0,017 | 1 | 1 | 140 | 0,06 | 95 | 75 |
| 4,4'-Isopropylidendi-cyclohexanol | 0,24 | 0,029 | 0,017 | 1 | 1 | 150 | 0,06 | 99 | 97 |
| 4-Hydroxycyclohexan-carbonsäureethylester | 0,18 | 0,029 | 0,017 | 2 | 0,6 | 130 | 0,06 | 82 | 95 |
| 2-Adamantanol | 0,15 | 0,058 | 0,035 | 1 | 0,6 | 150 | 0,06 | 99 | 98 |

| | | | | | | | | | |
|---|---|---|---|---|---|---|---|---|---|
| *1: Masse 12*-*Hydroxydodecansäuremethylester; 2: Masse [Carbonylchlorohydridotris-(triphenylphosphan)ruthenium(II)]; 3: Masse Xantphos; 4: Volumen Lösungsmittel; 5: Masse Ammoniak; 6: Reaktionstemperatur; 7: Verhältnis des Flüssigphasenvolumens zum Gasphasenvolumen; 8: Umsatz 12*-*Hydroxydodecansäuremethylester; 9: Selektivität zur 12*-*Aminododecansäuremethylester*. | | | | | | | | | |

## Patentansprüche

1. Verfahren zur Herstellung von primären Aminen umfassend die Verfahrensschritte
A) Bereitstellung einer Lösung eines Alkohols in einer fluiden, nicht gasförmigen Phase,
B) in Kontakt Bringen der Phase mit freiem Ammoniak und/oder mindestens einer Ammoniak freisetzenden Verbindung und eines homogenen Katalysatorsystems enthaltend mindestens einen Xantphos-Liganden der allgemeinen Formel 1 und eine Übergangsmetallverbindung 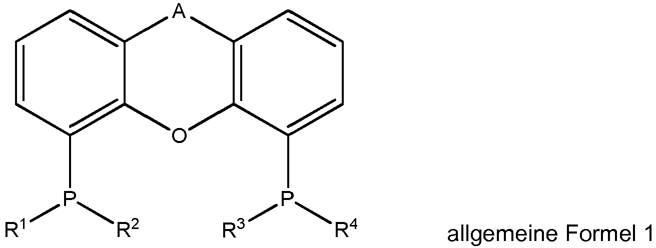 wobei R¹, R², R³ und R⁴ unabhängig voneinander gleich oder verschieden ausgewählt aus der Gruppe enthaltend Phenyl, tert. Butyl, Isopropyl, und A ausgewählt aus der Gruppe enthaltend -C(CH₃)₂-, -CH₂CH₂-, -Si(CH₃)₂-, -S-, -O-,-C(C(CH₃)₂)- und 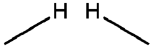 und gegebenenfalls
C) Isolierung des in Verfahrensschritt B) gebildeten primären Amins,
**dadurch gekennzeichnet,**
**dass** in Verfahrensschritt B) der Katalysator Carbonylchlorohydrido-[9,9-Dimethyl-4,5-bis(diphenylphosphino)-xantheno] ruthenium(II)] eingesetzt wird.

2. Verfahren gemäß Anspruch 1,
**dadurch gekennzeichnet,**
**dass** der in Verfahrensschritt A) eingesetzte Alkohol ausgewählt ist aus der Gruppe aliphatischer, linearer ω-Hydroxycarbonsäuren mit einer Kohlenstoffkette umfassend mindestens 8 Kohlenstoffatome.

3. Verfahren gemäß mindestens einem der vorherigen Ansprüche,
**dadurch gekennzeichnet,**
**dass** der Alkohol in einer Konzentration von zwischen 0,1 und 1000 mmol/L, bevorzugt zwischen 0,1 und 100 mmol/L und besonders bevorzugt zwischen 0,1 und 10 mmol/L, bezogen auf die fluide Phase eingesetzt wird.

4. Verfahren gemäß mindestens einem der vorherigen Ansprüche,
**dadurch gekennzeichnet,**
**dass** in Verfahrensschritt B) flüssiger oder überkritischer Ammoniak und/oder eine Lösung von Ammoniumsalzen in einem Lösungsmittel eingesetzt werden.

5. Verfahren gemäß mindestens einem der vorherigen Ansprüche,
**dadurch gekennzeichnet,**
**dass** der der Ammoniak in Verfahrensschritt B) bezogen auf die Hydroxylgruppen im Alkohol in einem molaren Verhältnis von mindestens 5 zu 1, bevorzugt 50 zu 1, besonders bevorzugt 500 zu 1, eingesetzt wird.

6. Verfahren gemäß mindestens einem der vorherigen Ansprüche,
**dadurch gekennzeichnet,**
**dass** Verfahrensschritt B) bei einem Druck im Bereich zwischen 1 und 1000 bar, bevorzugt zwischen 5 und 500 bar und besonders bevorzugt zwischen 5 und 20 bar, durchgeführt wird.

7. Verfahren gemäß mindestens einem der vorherigen Ansprüche,
**dadurch gekennzeichnet,**
**dass** Verfahrensschritt B) in einem Temperaturbereich zwischen 80 und 220 °C, bevorzugt zwischen 100 und 200 °C und besonders bevorzugt zwischen 120 und 170 °C, durchgeführt wird.

8. Verfahren gemäß mindestens einem der vorherigen Ansprüche,
**dadurch gekennzeichnet,**
**dass** das Volumenverhältnis der Flüssigphase zu dem der Gasphase im Verfahrensschritt B größer oder gleich 0,05, bevorzugt größer 0,1, insbesondere größer 0,2 ist.

9. Verfahren gemäß mindestens einem der vorherigen Ansprüche,
**dadurch gekennzeichnet,**
**dass** es in Abwesenheit von Wasserstoff durchgeführt wird.

## Claims

1. Process for preparing primary amines, which comprises the process steps
A) provision of a solution of an alcohol in a fluid, nongaseous phase,
B) contacting of the phase with free ammonia and/or at least one ammonia-liberating compound and a homogeneous catalyst system containing at least one xantphos ligand of the general formula 1 and a transition metal compound 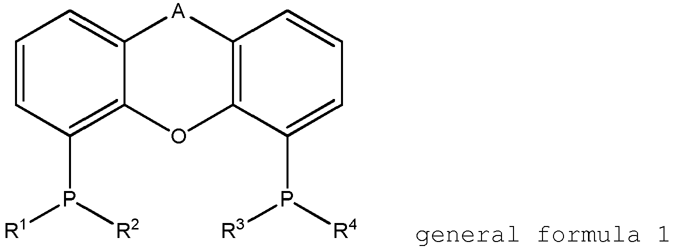 where R¹, R², R³ and R⁴ are identical or different and are selected independently from the group containing phenyl, tert-butyl and isopropyl, and A is selected from the group containing -C(CH₃)₂-, -CH₂CH₂-, -Si(CH₃)₂-, -S-, -O-, -C(C(CH₃)₂)- and 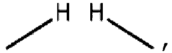 and optionally
C) isolation of the primary amine formed in process step B),
**characterized in that**
the catalyst carbonylchlorohydrido[9,9-dimethyl-4,5-bis(diphenylphosphino)xantheno]ruthenium(II)] is used in process step B).

2. Process according to Claim 1,
**characterized in that**
the alcohol used in process step A) is selected from the group consisting of aliphatic, linear ω-hydroxycarboxylic acids having a carbon chain comprising at least 8 carbon atoms.

3. Process according to at least one of the preceding claims,
**characterized in that**
the alcohol is used in a concentration of from 0.1 to 1000 mmol/l, preferably from 0.1 to 100 mmol/l and particularly preferably from 0.1 to 10 mmol/l, based on the fluid phase.

4. Process according to at least one of the preceding claims,
**characterized in that**
liquid or supercritical ammonia and/or a solution of ammonium salts in a solvent is/are used in process step B).

5. Process according to at least one of the preceding claims,
**characterized in that**
the ammonia is used in a molar ratio to the hydroxyl groups in the alcohol of at least 5:1, preferably 50:1, particularly preferably 500:1, in process step B).

6. Process according to at least one of the preceding claims,
**characterized in that**
process step B) is carried out at a pressure in the range 1 to 1000 bar, preferably from 5 to 500 bar and particularly preferably from 5 to 20 bar.

7. Process according to at least one of the preceding claims,
**characterized in that**
process step B) is carried out in the temperature range from 80 to 220°C, preferably from 100 to 200°C and particularly preferably from 120 to 170°C.

8. Process according to at least one of the preceding claims,
**characterized in that**
the volume ratio of the liquid phase to the gas phase in process step B is greater than or equal to 0.05, preferably greater than 0.1, in particular greater than 0.2.

9. Process according to at least one of the preceding claims,
**characterized in that**
it is carried out in the absence of hydrogen.

## Revendications

1. Procédé de fabrication d'amines primaires comprenant les étapes de procédé suivantes :
A) la préparation d'une solution d'un alcool dans une phase fluide non gazeuse,
B) la mise en contact de la phase avec de l'ammoniac libre et/ou au moins un composé libérant de l'ammoniac et un système catalytique homogène contenant au moins un ligand xantphos de formule générale 1 et un composé de métal de transition 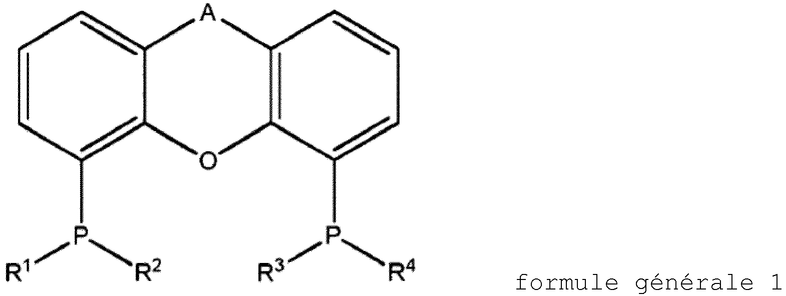 dans laquelle R¹, R², R³ et R⁴ sont choisis indépendamment les uns des autres de manière identique ou différente dans le groupe contenant phényle, tert.-butyle, isopropyle, et A est choisi dans le groupe contenant - C(CH₃)₂-, -CH₂CH₂-, -Si(CH₃)₂-, -S-, -O-, -C(C(CH₃)₂)- et 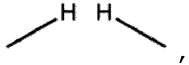 et éventuellement,
C) l'isolement de l'amine primaire formée à l'étape de procédé B),
**caractérisé en ce que**
à l'étape de procédé B), le catalyseur carbonylchlorohydrido-[9,9-diméthyl-4,5-bis(diphénylphosphino)-xanthéno]ruthénium(II)] est utilisé.

2. Procédé selon la revendication 1, **caractérisé en ce que** l'alcool utilisé à l'étape de procédé A) est choisi dans le groupe des acides ω-hydroxycarboxyliques aliphatiques linéaires ayant une chaîne carbonée comprenant au moins 8 atomes de carbone.

3. Procédé selon au moins l'une quelconque des revendications précédentes, **caractérisé en ce que** l'alcool est utilisé en une concentration comprise entre 0,1 et 1 000 mmol/l, de préférence comprise entre 0,1 et 100 mmol/l et de manière particulièrement préférée comprise entre 0,1 et 10 mmol/l, par rapport à la phase fluide.

4. Procédé selon au moins l'une quelconque des revendications précédentes, **caractérisé en ce qu'**à l'étape de procédé B), de l'ammoniac liquide ou supercritique et/ou une solution de sels d'ammonium dans un solvant sont utilisés.

5. Procédé selon au moins l'une quelconque des revendications précédentes, **caractérisé en ce que** l'ammoniac est utilisé à l'étape de procédé B) par rapport aux groupes hydroxyle dans l'alcool en un rapport molaire d'au moins 5 sur 1, de préférence de 50 sur 1, de manière particulièrement préférée de 500 sur 1.

6. Procédé selon au moins l'une quelconque des revendications précédentes, **caractérisé en ce que** l'étape de procédé B) est réalisée à une pression dans la plage comprise entre 1 et 1 000 bar, de préférence entre 5 et 500 bar et de manière particulièrement préférée entre 5 et 20 bar.

7. Procédé selon au moins l'une quelconque des revendications précédentes, **caractérisé en ce que** l'étape de procédé B) est réalisée dans une plage de température comprise entre 80 et 220 °C, de préférence entre 100 et 200 °C et de manière particulièrement préférée entre 120 et 170 °C.

8. Procédé selon au moins l'une quelconque des revendications précédentes, **caractérisé en ce que** le rapport volumique entre la phase liquide et la phase gazeuse à l'étape de procédé B est supérieur ou égal à 0,05, de préférence supérieur à 0,1, notamment supérieur à 0,2.

9. Procédé selon au moins l'une quelconque des revendications précédentes, **caractérisé en ce qu'**il est réalisé en l'absence d'hydrogène.
